# EUROPÄISCHE PATENTSCHRIFT

(11) **EP 1 110 082 B1**
(45) Veröffentlichungstag und Bekanntmachung des Hinweises auf die Patenterteilung: **23.11.2005**
(21) Anmeldenummer: 99944400.3
(22) Anmeldetag: 14.08.1999
(51) Int. Cl.: G01N 33/15

(54) **MINI-BASKET ZUR UNTERSUCHUNG DER WIRKSTOFFFREISETZUNG AUS EINER ARZNEIFORM**
MINI-BASKET FOR ANALYZING ACTIVE SUBSTANCE RELEASE FROM A MEDICAMENT FORM
MINI-PANIER POUR ANALYSER LA LIBERATION D'UN PRINCIPE ACTIF HORS D'UNE FORME GALENIQUE

(30) Priorität: 29.08.1998 DE 19839398
(43) Veröffentlichungstag der Anmeldung: 27.06.2001
(73) Patentinhaber: Sanofi-Aventis Deutschland GmbH, 65929 Frankfurt am Main (DE)
(72) Erfinder: LOOS, Petra, D-65779 Kelkheim (DE); HORLE, Brigitte, D-65931 Frankfurt am Main (DE); MERKEL, Rüdiger, D-Hochheim (DE)
(86) Internationale Anmeldenummer: PCT/EP1999/005980
(87) Internationale Veröffentlichungsnummer: WO 2000/013012

(56) Entgegenhaltungen:
- DE-A- 3 715 961
- FR-A- 2 160 193
- GB-A- 2 136 123
- US-A- 3 802 272
- US-A- 4 856 909
- US-A- 5 011 662

## Beschreibung

Im Folgenden wird eine Vorrichtung zur Untersuchung der in vitro Wirkstofffreisetzung aus einer festen Arzneiform bestehend aus einem neuen Mini-Basket und deren Verwendung beschrieben.

Während der Entwicklung einer Arzneiform werden Qualität, Wirksamkeit und Unbedenklichkeit des Arzneimittels u.a. durch in vivo und in vitro Untersuchungen geprüft. Dabei kommt den in vitro Untersuchungen besondere Bedeutung zu, da sie oftmals in der Lage sind, kleinere Veränderungen der Arzneiform, die Auswirkungen auf die Wirksamkeit bzw. Unbedenklichkeit (und damit auf die Arzneimittelsicherheit) haben können, aufzuzeigen. Durch in vitro Freisetzungsuntersuchungen kann die galenische Formulierung unter Reduktion aufwendiger kosten- und zeitintensiver in vivo Studien optimiert und die Qualität der hergestellten Chargen während der Entwicklung, der Lagerung und der Produktion kontrolliert werden (Kommentar zum DAB 1996, V.5.4. Wirkstofffreisetzung aus festen oralen Arzneiformen, Govi-Verlag, Herausgeber Hartke, Hartke, Mutschler, Rücker, Wichtl). Ein Vergleich der erhaltenen in vitro Daten mit in vivo Studien kann zu einer Reduktion der Prüfungen an Menschen bzw. Tieren führen, da Rückschlüsse auf das in vivo Verhalten bei späteren Mustern möglich sind.

Eine wesentliche Voraussetzung für die Wirkstoffresorption und damit für die Bioverfügbarkeit ist die Wirkstofffreisetzung aus der Arzneiform (Kommentar zum DAB 1996, V.5.4. Wirkstofffreisetzung aus festen oralen Arzneiformen, Govi-Verlag, Herausgeber Hartke, Hartke, Mutschler, Rücker, Wichtl). Die Arzneibücher beschreiben zu diesem Zweck einige offizielle in vitro Freisetzungsuntersuchungsmethoden mit den dazugehörigen bekannten Apparaturen. So wird zur Bestimmung der Freisetzung von Wirkstoffen aus festen Arzneiformen - wie Tabletten, Kapseln, Pellets, oder Zäpfchen - die Blattrührer-, Drehkörbchen- oder Durchflußzellen-Apparatur verwendet. Erstere sind geschlossene Systeme, bei denen sich die zu prüfende Arzneiform entweder in dem zur Apparatur gehörenden zylindrischen Gefäß oder im Drehkörbchen selbst befindet und der Blattrührer bzw. das Drehkörbchen der Agitation dienen. Die Durchflußzellen-Apparatur kann als geschlossenes (Rückfuhr des Freisetzungsmediums) oder offenes System (Zufuhr frischen Freisetzungsmediums) verwendet werden. Zu festgelegten Zeitpunkten wird Prüfflüssigkeit entnommen und der darin gelöste Arzneistoff bestimmt. Diese genannten Apparaturen, Blattrührer-Apparatur, Drehkörbchen-Apparatur und Durchflußzelle, sind aus dem Europäischen Arzneibuch 1997, Govi-Verlag, Seiten 136 bis 139 oder auch aus dem Amerikanischen Arzneibuch, The United States Pharmacopoeial Convention Inc., Twinbrook Parkway, Rockville, MD, Seiten 1791 bis 1799 (USP 23/NF 18) bekannt.

Beispielsweise beschreibt US Patent 4856909A eine Vorrichtung aus Drahtgewebe zur Bestimmung der Auflösungsgeschwindigkeit von Tabletten o.ä.. Diese besteht aus einem Körbchen zur Aufnahme der Tabletten und einen Deckel mit Befestigungsklammern, wobei der Deckel über einen Stab fest mit einer Antriebseinheit verbunden ist, wobei der Stab als Antriebswelle dient. Das Körbchen kann hierbei in horizontaler und vertikaler Richtung bewegt werden.

US Patent 5011662A bzw. die äquivalente britische Anmeldung GB-A-2136123 beschreiben eine Vorrichtung zur Durchführung von Auflosungstests, wobei die in den jeweils lösemittel-durchlässigen Behältnissen befindlichen Proben in jeweils einzelne Lösungsmittelbehälter eingebracht werden. Diese Vorrichtung erlaubt u.a. den einfachen Vergleich der Auflösung identischer Proben in unterschiedlichen Losungsmitteln.

DE-A-3715961 beschreibt eine Vorrichtung zur Messung der Zerfallsgeschwindigkeit von Tabletten, Dragees oder Kapseln in definierten Lösungen unter kontrollierten und konstanten Bedingungen. Die Vorrichtung umfasst neben einer motorisch angetriebenen und elektronisch gesteuerten Hubvorrichtung zum Auf- und Abbewegen des Probengefäßes eine weitere Hubvorrichtung zum vollständigen Aus- und Einfahren des Probengefäßes aus dem Lösungsmittelbehälter und einen Antrieb zur Durchführung einer elektronisch steuerbaren Relativbewegung zwischen den Hubvorrichtungen und dem Lösungsmittelbad. Auf diese Weise wird für das Probengefäß auf einfache und reproduzierbare Art der Wechsel des Lösungsmittels ermöglicht.

US-A-3802272 beschreibt eine Apparatur zur automatischen Bestimmung der Lösungsgeschwindigkeit einer Vielzahl von Feststoffen in Flüssigkeiten. Die Apparatur umfasst u.a. mehrere Lösungskammem, die über jeweils zugehörige Ventile und Rohrleitungen mit einem Analysegerät verbunden sind.

FR-A-2160193 beschreibt eine Vorrichtung zur Untersuchung der Auflösungsgeschwindigkeit fester Proben in einem Lösungsmittel in einem geschlossenen Umlaufsystem. Die Vorrichtung umfasst des weiteren einen drehbaren Hohlfilter sowie Mittel zur Probenentnahme aus dem Inneren des Filters.

Die im Stand der Technik beschriebenen Vorrichtungen erlauben keine schnelle und einfache Entnahme des Probengefäßes aus dem Lösungsmittel und Verbringung desselben in eine zweite, unterschiedlich geartete Messvorrichtung. Hier will die vorliegende Erfindung durch die in den vorliegenden Ansprüchen bezeichneten Ausführungsformen Abhilfe schaffen, wie es beispielsweise die Messung von Arzneiformen mit modifizierter Wirkstofffreisetzung erfordert.

Arzneiformen mit modifizierter Wirkstofffreisetzung sind überzogene oder nichtüberzogene Arzneiformen, bei denen die Freisetzungsgeschwindigkeit oder der Ort der Freisetzung gezielt verändert wird. Daneben gibt es noch magensaft-resistente Arzneiformen, die im Magensaft beständig sind und den Wirkstoff im Darmsaft freisetzen. Sofern der Wirkstoff im Freisetzungsmedium gut löslich ist, können diese Arzneiformen mit den zuvor erwähnten Apparaturen (Blattrührer- und Drehkörbchen-Apparatur) nach entsprechendem Validierungsaufwand geprüft werden. Eventuell wird dabei auch die Durchflußzelle zu Hilfe genommen, besonders wenn Profile der Freisetzung aufgenommen werden sollen. Die sogenannten Sink-Bedingungen sollten während des Versuches eingehalten werden, d.h. die Konzentration des zu prüfenden Wirkstoffs im Freisetzungsmedium sollte 30% der Sättigungskonzentration nicht überschreiten.

Die Untersuchung der in vitro Wirkstofffreisetzung von Arzneiformen mit im Freisetzungsmedium schwerlöslichen Arzneistoffen kann im Gegensatz zu den vorherigen Ausführungen in der bekannten Blattrührer- und Drehkörbchen-Apparatur problematisch werden, da aufgrund des begrenzten Volumens die Freisetzung von der Löslichkeit des Wirkstoffs gesteuert sein kann und nicht von der Freigabe aus der Arzneiform. Abhilfe kann in diesem Fall die bekannte Durchflußzelle (als offenes System) schaffen, die kontinuierlich frisches Freisetzungsmedium nachführt. Sofern die den schwerlöslichen Wirkstoff enthaltene Arzneiform nicht magensaft-resistent überzogen ist, ist die Verwendung der Durchflußzelle eine elegante Methode, um Freisetzungsprofile von Arzneiformen mit modifizierter Wirkstofffreisetzung aufzuzeigen. Magensaft-resistent überzogene Arzneiformen müssen allerdings vor der eigentlichen Prüfung auf Wirkstofffreisetzung auf die Integrität des Überzuges geprüft werden, d.h. vorgeschaltet ist ein Untersuchungsschritt in einem sauren Freisetzungsmedium. Soll die Untersuchung mit der Durchflußzelle erfolgen, kann innerhalb der ersten gesammelten Fraktionen ein pH-Gefälle entstehen, das die Ergebnisse verfälschen kann.

Bei einer magensaft-resistenten Arzneiform kann es sich z.B. um eine Kapsel handeln, die den Wirkstoff eingeschlossen in sogenannten Pellets enthält. Diese Pellets können magensaft-resistent überzogen sein, d.h. sie sollen den Wirkstoff erst bei höheren pH-Werten freisetzen, wie sie während der Passage durch den Darmtrakt vorliegen. In der Konsequenz sind diese Pellets bei ansteigenden pH-Werten immer weniger stabil, d.h. sie setzen den Wirkstoff in Freisetzungsmedien mit höheren pH-Werten relativ schnell frei. Eine Diskriminierungsfähigkeit zwischen kleineren Formulierungsunterschieden kann unter Umständen nicht mehr möglich sein. Ist gleichzeitig der Wirkstoff in Medien mit niedrigeren pH-Werten nur schwer löslich, weist in diesem Fall das Ergebnis nicht auf die Freisetzung aus der Arzneiform hin, sondern ist gesteuert durch die Löslichkeit des Wirkstoffs. Ein Freisetzungsmedium mit hohem pH-Wert kann dieses zwar umgehen, kann aber aufgrund der zuvor geschilderten Schwierigkeit (geringe Diskriminierungsfähigkeit) nicht verwendet werden.

Ein Freisetzungsmedium mit mittlerem pH-Wert, angewendet in der bekannten Durchflußzellen-Apparatur, kann einerseits zwischen unterschiedlichen Formulierungen diskriminieren, anderseits auch durch Zufuhr frischen Mediums unter Umgehung der Löslichkeitsproblematik eine Freisetzungsuntersuchung ermöglichen. Aus DE 29 42 129 A1 ist ein Verfahren bekannt, bei dem die Auflösekammem von mindestens zwei Durchflußzellen miteinander verbunden sind. Damit soll erreicht werden, daß ein gesteuerter Übertritt von Auflösemedium mit Arzneistoffpartikeln oder Arzneiformen von einer Zelle zur nächsten möglich ist. Als Schwierigkeit erweist sich in diesem speziellen Fall allerdings die Untersuchung der Magensaftresistenz. Aufgrund des pH-Gefälles in der ersten basischen Fraktion nach zuvor erfolgter Durchführung mit einem sauren Medium (saure Restflüssigkeit befindet sich zwangsweise noch im Schlauchsystem der Apparatur) fällt der Wirkstoff aus und kann nicht vollständig analysiert werden.

Ziel der Erfindung ist es daher, eine Vorrichtung zur Untersuchung der in vitro Wirkstofffreisetzung zu entwickeln, mit der diese Nachteile ausgeräumt werden.

Gegenstand der Erfindung ist eine Vorrichtung zur in vitro Wirkstofffreisetzung aus einer festen Arzneiform bestehend aus einem Mini-Basket mit einem unteren Teil und einem oberen Teil, dadurch gekennzeichnet, daß der untere Teil (Körbchen) und der obere Teil (Deckel) des Mini-Baskets aus Drahtgewebe bestehen und der obere Teil (Deckel) an der Außenseite einen Griff aufweist und besagte Vorrichtung in eine Durchflußzelle einpaßbar ist.

Es werden feste Arzneiformen, wie z.B. Tabletten, Kapseln, Pellets, Zäpfchen, oder bevorzugt magensaft-resistente Arzneiformen der Untersuchung unterworfen. Der Deckel wird auf der Innenseite durch eine oder mehrere, bevorzugt eine bis drei, besonders bevorzugt eine Befestigungsklammer oder andere Vorrichtungen formschlüssig auf der Oberseite des Körbchens festgehalten, u.a. damit das Mini-Basket bündig in die bekannten Durchtlußzellen (Typ A und Typ B (siehe Europäisches Arzneibuch 1997, Govi-Verlag, Seiten 136 - 139 bzw. auch andere Arzneibücher) eingepaßt werden kann. Die Befestigungsklammer ist z.B. auf der Innenseite des Deckels mittig angebracht, z.B. angeschweißt, ohne das Drahtgewebe zu verkleinern. Auch andere Befestigungsklammern aus Metall sind möglich, beispielsweise am Rand des Deckels angebracht oder Befestigungsklammern ohne Mittelstück. Das Körbchen aus verschweißtem Drahtgewebe ist zu einem Zylinder geformt, dessen Ober- und Unterkante von einem schmalen Metallband umschlossen sind. Das Drahtgewebe, das z.B. aus rostfreiem Stahl besteht, kann - je nach Prüfflüssigkeit - auch mit einem geeigneten Material (z.B. Gold) beschichtet sein, um zu gewährleisten, daß diese Teile nicht mit der zu testenden Zubereitung oder der Prüfflüssigkeit reagieren oder das Verhalten beeinflussen. Die Ausrichtung des Drahtgewebes ist beliebig, d.h. vertikal/horizontal oder auch diagonal, bevorzugt jedoch vertikal/horizontal.

Die Abmaße des Mini-Baskets können je nach der gewählten in vitro Freisetzungsuntersuchung variieren. Nachfolgende Angaben entsprechen ungefähren Werten. Die Höhe ist generell frei wählbar, je nach Durchflußzelle beträgt die max. Höhe des Mini-Baskets 35 bzw. 50 mm. So kommen beispielsweise in Frage Körbchen mit einer Höhe von L (Gesamthöhe) = 10 mm bis 40 mm, bevorzugt = 20 mm, L₁ (Höhe des Drahtgewebes) bevorzugt = 16 mm, L₂ (Höhe des Metallbandes oben) bevorzugt = 1 mm, L₃ (Höhe des Metallbandes unten) bevorzugt = 3 mm und einem Durchmesser von D (Gesamtdurchmesser des Bodens) = 11,5 mm bis 22,6 mm, bevorzugt 11,9 mm (Durchflußzelle Typ B) und 22,5 mm (Durchflußzelle Typ A), besonders bevorzugt = 22,5 mm (d.h. bündig entsprechend dem Durchmesser der gewählten Durchflußzelle), D₁ (Durchmesser des Drahtgewebes) bevorzugt = 16,5 mm, D₂ (Durchmesser/Breite des Metallbandes) bevorzugt = 3 mm, ferner Deckel deren Durchmesser dem des Körbchens und deren Höhe der Breite des Metallbandes L₃ (Höhe des Metallbandes) bevorzugt = 3 mm entspricht. Die Größe und das Material des Griffs kann verschieden sein, steht jedoch im Einklang mit der/dem des Mini-Baskets. Die Art des Griffs ist so ausgestaltet, daß das Mini-Basket leicht aus der in vitro Wirkstofffreisetzungsapparatur herausgehoben werden kann. Bespielweise kommen Bügel, Henkel, Knopf, Stab oder Öse in Frage, aber auch eine Kombination Stab - Öse ist denkbar, bevorzugt ist jedoch der Bügel. Die Befestigung des Griffs am Deckel, mittig oder auch am Deckelrand (Metallband), sollte möglichst ohne Verkleinerung der Drahtgewebefläche erfolgen. Der Griff kann beispielsweise am Deckel angeschweißt, angeschraubt oder angenietet sein, wobei letztere Befestigungsformen auch die Befestigungsklammer mit einbeziehen kann.

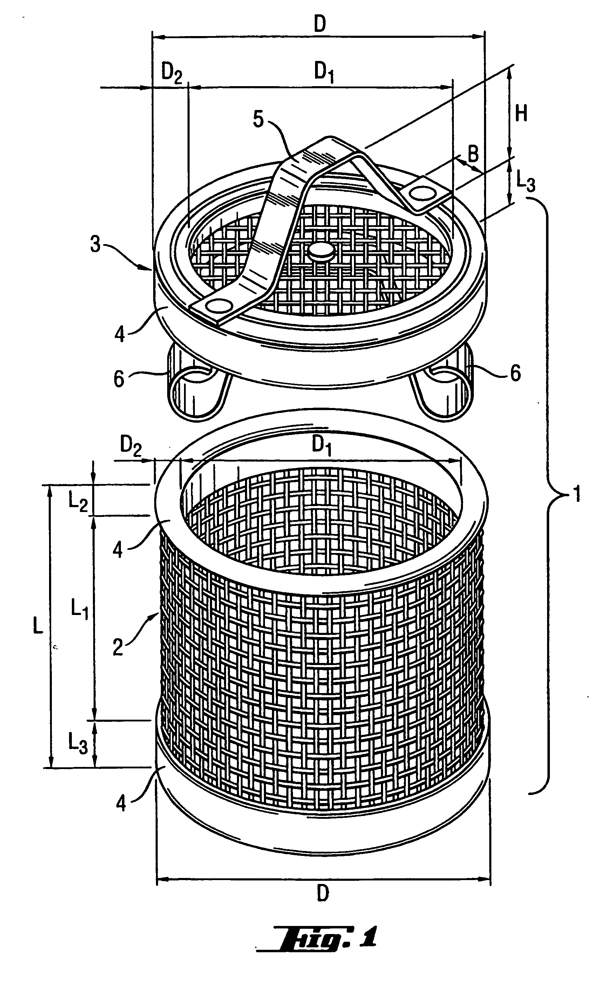

Ein Ausführungsbeispiel der Erfindung ist in Figur 1 dargestellt und wird im folgenden näher beschrieben:

Das Mini-Basket (1), dessen Abmaße bevorzugt so gewählt sind (siehe vorletzter Absatz), daß es je nach zu verwendender in vitro Freisetzungsuntersuchung in die entsprechenden Apparaturen (z.B. Blattrührer-Apparatur und/oder Durchflußzellen Typ A oder Typ B) bündig eingebracht werden kann, besteht aus einem unteren Teil (Körbchen) (2) und aus einem oberen Teil (Deckel) (3), deren Abmessungen durch die gewählte(n) in vitro Freisetzungsmethode(n) und der/die damit verbundene(n) Größe(n) der Apparaturen bestimmt sind. Das Körbchen (2) ist aus verschweißtem Drahtgewebe und zylinderförmig ausgebildet. Die Drahtstärke des Drahtgewebes (d) kann beispielsweise 0,1 bis 0,3 mm, bevorzugt 0,2 bis 0,3 mm und besonders bevorzugt 0,254 mm betragen. Die lichte Maschenweite ist in Abhängigkeit von der zu untersuchenden Arzneiform von 0,1 mm bis zum Durchmesser der zu untersuchenden Partikel wählbar, bevorzugt 0,2 bis 1 mm, besonders bevorzugt 0,55 mm. Ober- und Unterkante des Körbchens (2) und die Kante des Deckels (3) sowie die Ränder der Bodenplatte des Körbchens und der Oberseite des Deckels sind von einem schmalen Metallband (4), vorzugsweise aus demselben Material wie das Drahtgewebe, umschlossen. Der Rand des Deckels (3) schließt mit dem oberen Rand des Körbchens (2) bündig ab. Zusätzlich werden Deckel (3) und Körbchen (2) durch eine Befestigungsklammer (6), die u.a. die Breite des Metallbandes haben kann, formschlüssig zusammengehalten, ohne daß diese das Drahtgewebe verkleinern. Der Deckel (3) ist mit einem Bügel (5) aus demselben Material versehen. Der Bügel dient zum Herausheben des Mini-Baskets aus der in vitro Wirkstofffreisetzungsapparatur. Die Maße des am Deckel befestigten Bügels oder auch der anderen Griffe können beliebig variieren, bevorzugt ist im Fall des Bügels eine Höhe von 4 mm und eine Breite von 2 mm.

Gegenstand der Erfindung ist weiterhin ein Verfahren zur in vitro Wirkstofffreisetzung aus einer festen Arzneiform (z.B. Tabletten, Kapseln, Pellets), bevorzugt magensaftresistenter Pellets, durch Prüfung in saurem Freisetzungsmedium und anschließend bei höherem pH, das dadurch gekennzeichnet ist, daß man ein die feste Arzneiform enthaltenes Mini-Basket, wie oben beschrieben, in ein Gefäß einer bekannten Blattrührer-Apparatur gibt und im sauren Freisetzungsmedium auf Integrität des Überzugs prüft und anschließend das Mini-Basket mit Hilfe des sich am Deckel befindlichen Griffs aus dem Gefäß der bekannten Blattrührer-Apparatur hebt, in eine bekannte Durchflußzelle einbringt und die Freisetzung des Wirkstoffs aus der Arzneiform bei höherem pH testet. Das Körbchen ist so konstruiert, daß es bündig in die Zelle paßt und das Prüfmedium durch die Maschen strömen kann.

Eine weitere Ausgestaltung des Mini-Baskets unterscheidet sich durch einen auf die Abmessungen des Körbchens modifizierten oberen Teil (Deckel) in Form einer Platte, an die ein Stab befestigt (z.B. angeschweißt) ist und die ein oder mehrere, bevorzugt ein bis drei Befestigungsklammern besitzt, die das Körbchen festhalten und eine Rotation konzentrisch zur Achse des Mini-Baskets ermöglichen, vergleichbar der im Europäischen Arzneibuch 1997, Govi-Verlag, Seite 137 beschriebenen Drehkörbchen-Apparatur. Dabei entspricht das neuartige Mini-Basket mit Ausnahme des Deckels der zuvor angegebenen Beschreibung. Ein Betrieb ähnlich der bekannten Drehkörbchen-Apparatur ist damit möglich.

Gegenstand der Erfindung ist daher ein Verfahren zur in vitro Wirkstofffreisetzung aus einer festen Arzneiform durch Prüfung in saurem Freisetzungsmedium und anschließend bei höherem pH, dadurch gekennzeichnet, daß man ein die feste Arzneiform enthaltenes Mini-Basket mit Stab, wie im letzten Absatz beschrieben, in ein Gefäß einer bekannten Drehkörbchen-Apparatur gibt und im sauren Freisetzungsmedium auf Integrität des Überzugs prüft und anschließend das Mini-Basket aus dem Gefäß der Drehkörbchen-Apparatur hebt, den Deckel in Form einer Platte mit Stab durch einen entsprechenden Drahtgewebe-Deckel mit Griff, wie oben beschrieben, ersetzt, mit Hilfe des Griffs in eine bekannte Durchflußzelle einbringt und die Freisetzung des Wirkstoffs aus der Arzneiform bei höherem pH testet. Somit ist es möglich, daß die zu untersuchende Arzneiform ebenfalls in der Drehkörbchen-Apparatur und anschließend in der Durchflußzelle zu untersuchen.

Unter Verwendung des Mini-Baskets können also die Vorteile unterschiedlicher Systeme zur Prüfung der in vitro Wirkstofffreisetzung aus komplizierteren festen oralen Arzneiformen genutzt werden. In einem Analysengang kann die Wirkstofffreisetzung durchgeführt werden, deren Ergebnisse die Arzneimittelsicherheit erhöhen kann.

## Patentansprüche

1. Vorrichtung zur in vitro Wirkstofffreisetzung aus einer festen Arzneiform bestehend aus einem Mini-Basket(1) mit einem unteren Teil und einem oberen Teil, wobei der untere Teil (Körbchen(2)) und der obere Teil (Deckel(3)) des Mini-Baskets(1) aus Drahtgewebe(d) bestehen und der obere Teil (Deckel(3)) an der Außenseite einen Griff aufweist und besagte Vorrichtung in eine Durchflusszelle einpaßbar ist

2. Vorrichtung gemäß Anspruch 1, **dadurch gekennzeichnet, daß** der untere Teil (Körbchen(2)) über eine oder mehrere Befestigungsklammem(6) mit dem oberen Teil (Deckel(3)) verbunden ist.

3. Vorrichtung gemäß den Ansprüchen 1 bis 2, **dadurch gekennzeichnet, daß** der untere Teil (Körbchen(2)) über eine Befestigungsklammer(6) mit dem oberen Teil (Deckel(3)) verbunden ist.

4. Vorrichtung gemäß den Ansprüchen 1 bis 3, **dadurch gekennzeichnet, daß** die Entnahmevorrichtung ein Bügel(5) ist.

5. Verwendung einer Vorrichtung gemäß den Ansprüchen 1 bis 4 in einer Blattrührer-Apparatur und/oder Durchflußzelle.

6. Verfahren zur in vitro Wirkstofffreisetzung aus einer festen Arzneiform durch Prüfung In saurem Freisetzungsmedium und anschließend bei höherem pH, **dadurch gekennzeichnet, daß** man ein die feste Arzneiform enthaltenes Mini-Basket(1) der Ansprüche 1 bis 4 in ein Gefäß einer bekannten Blattrührer-Apparatur gibt und im sauren Freisetzungsmedium auf Integrität des Überzugs prüft und anschließend das Mini-Basket(1) mit Hilfe des sich am Deckel befindlichen Griffs aus dem Gefäß der bekannten Blattrührer-Appäratur hebt, in eine bekannte Durchflußzelle einbringt und die Freisetzung des Wirkstoffs aus der Arzneiform bei höherem pH testet.

7. Verfahren zur in vitro Wirkstofffreisetzung aus einer festen Arzneiform durch Prüfung in saurem Freisetzungsmedium und anschließend bei höherem pH, **dadurch gekennzeichnet, daß** man ein die feste Arzneiform enthaltenes Mini-Basket(1) bestehend aus einem unteren Teil (Körbchen(2)) aus Drahtgewebe(d) und einem oberen Teil (Deckel) in Form einer Platte, an die ein Stab befestigt ist und die ein oder mehrere Befestigungsklammern besitzt, die das Körbchen(2) und den Deckel(3) formschlüssig zusammenhalten und eine Rotation konzentrisch zur Achse des Mini-Baskets(1) ermöglichen, in ein Gefäß einer bekannten Drehkörbchen-Apparatur gibt und im sauren Freisetzungsmedium auf Integrität des Überzugs prüft und anschließend das Mini-Basket (1) aus dem Gefäß der Drehkörbchen-Apparatur hebt, den Deckel in Form einer Platte durch einen entsprechenden Drahtgewebe-Deckel(3) mit Griff ersetzt, in eine bekannte Durchflußzelle einbringt und die Freisetzung des Wirkstoffs aus der Arzneiform bei höherem pH testet.

## Claims

1. Device for in vitro active substance release from a solid medicament form, consisting of a mini-basket (1) with a bottom part and a top part, the bottom part (mini-basket (2)) and the top part (lid (3)) of the mini-basket (1) being made of wire screen fabric (d), and the top part (lid (3)) having a handle on the outer side, said device being able to be fitted into a continuous flow cell.

2. Device according to Claim 1, **characterized in that** the bottom part (mini-basket (2)) is connected to the top part (lid (3)) by one or more fixing clips (6).

3. Device according to Claims 1 to 2, **characterized in that** the bottom part (mini-basket (2)) is connected to the top part (lid (3)) by one fixing clip (6).

4. Device according to Claims 1 to 3, **characterized in that** the removal device is a bracket (5).

5. Use of a device according to Claims 1 to 4 in a paddle agitator and/or continuous flow cell.

6. Method for in vitro active substance release from a solid medicament form by testing in acid release medium and subsequently at a higher pH, **characterized in that** a mini-basket (1) according to Claims 1 to 4, containing the solid medicament form, is placed in a vessel of a known paddle agitator, the integrity of the coating is tested in the acid release medium, the mini-basket (1) is then lifted out of the vessel of the known paddle agitator with the aid of the handle located on the lid, and it is introduced into a known continuous flow cell, and the release of the active substance from the medicament form is tested at a higher pH.

7. Method for in vitro active substance release from a solid medicament form by testing in acid release medium and subsequently at a higher pH, **characterized in that** a mini-basket (1) containing the solid medicament form and consisting of a bottom part (mini-basket (2)) of wire screen fabric (d) and a top part (lid) in the form of a plate, to which a rod is attached and which has one or more fixing clips holding the mini-basket (2) and the lid (3) together frictionally and permitting a rotation concentric to the axis of the mini-basket (1), is placed in a vessel of a known rotating basket apparatus and the integrity of the coating is tested in the acid release medium, after which the mini-basket (1) is lifted out of the vessel of the rotating basket apparatus, the lid in the form of a plate is replaced by a corresponding wire screen fabric lid (3) with handle and introduced into a known continuous flow cell, and the release of the active substance from the medicament form is tested at a higher pH.

## Revendications

1. Dispositif de libération in vitro d'un principe actif d'une forme galénique solide d'un médicament, constitué d'un mini-panier (1) qui présente une partie inférieure et une partie supérieure, la partie inférieure (fond (2)) et la partie supérieure (couvercle (3)) du mini-panier (1) étant constituées d'un treillis de fils (d), la partie supérieure (couvercle (3)) présentant une poignée sur le côté extérieur et ledit dispositif pouvant être ajusté dans une cellule d'écoulement.

2. Dispositif selon la revendication 1, **caractérisé en ce que** la partie inférieure (fond (2) du panier) est reliée par une ou plusieurs pinces de fixation (6) à la partie supérieure (couvercle (3)).

3. Dispositif selon les revendications 1 à 2, **caractérisé en ce que** la partie inférieure (fond (2) du panier) est reliée par des pinces de fixation (6) à la partie supérieure (couvercle (3)).

4. Dispositif selon les revendications 1 à 3, **caractérisé en ce que** le dispositif de prélèvement est un étrier (5).

5. Utilisation d'un dispositif selon les revendications 1 à 4 dans un appareil d'agitation à pales et/ou dans une cellule d'écoulement.

6. Procédé de libération in vitro d'un principe actif d'une forme galénique solide d'un médicament par test dans un fluide acide de libération et ensuite à pH plus élevé, **caractérisé en ce que** l'on place un mini-panier (1) selon les revendications 1 à 4, qui contient la forme galénique solide d'un médicament, dans un récipient d'un appareil connu d'agitation à pales, que l'on vérifie l'intégrité de son revêtement dans le fluide acide de libération et qu'ensuite, à l'aide de la poignée située sur le couvercle, on retire le mini-panier (1) du récipient de l'appareil connu d'agitation à pales, on l'installe dans une cellule d'écoulement connue et on teste à un pH plus élevé la libération du principe actif par la forme galénique de médicament.

7. Procédé de libération in vitro du principe actif d'une forme galénique solide de médicament par test dans un fluide acide de libération et ensuite à pH plus élevé, **caractérisé en ce que** l'on place dans le récipient d'un appareil connu à panier rotatif un mini-panier (1) qui contient la forme galénique solide de médicament et qui est constitué d'une partie inférieure (fond (2) du panier) en treillis de fils (d) et d'une partie supérieure (couvercle) qui présente la forme d'une plaque à laquelle est fixée une barre et qui possède une ou plusieurs pinces de fixation qui maintiennent ensemble en correspondance géométrique le fond (2) du panier et le couvercle (3) et qui permettent une rotation concentrique à l'axe du mini-panier (1), que l'on vérifie l'intégrité du revêtement dans le fluide acide de libération et qu'ensuite on retire le mini-panier (1) du récipient de l'appareil à panier rotatif, qu'on remplace le couvercle en forme de plaque par un couvercle (3) correspondant en treillis de fils doté d'une poignée, qu'on le place dans une cellule d'écoulement connue et que l'on teste à un pH plus élevé la libération du principe actif par la forme galénique du médicament.
